# EUROPÄISCHE PATENTANMELDUNG

(11) **EP 2 907 481 A1**
(43) Veröffentlichungstag der Anmeldung: **19.08.2015**
(21) Anmeldenummer: 15450005.2
(22) Anmeldetag: 28.01.2015
(51) Int. Cl.: A61F 2/44

(54) **Implantat zum Einsetzen zwischen Wirbelkörper der Wirbelsäule und Bausatz zur Herstellung eines derartigen Implantats**

(30) Priorität: 12.02.2014 AT 602014
(71) Anmelder: HO-MED Handelsgesellschaft m.b.H., 1220 Wien (AT)
(72) Erfinder: Sabitzer, Ronald J., 1060 Wien (AT); Holzer, Gerald, 1190 Wien (AT)
(74) Vertreter: Miksovsky, Alexander

(57) **Zusammenfassung**

Bei einem Implantat (3) zum Einsetzen zwischen Wirbelkörper (1, 2) der Wirbelsäule, wobei das Implantat (3) eine gewölbte Oberfläche an seiner Oberseite (5) aufweist und in implantiertem Zustand an seiner Unterseite (4) unbeweglich mit dem daran anschließenden Wirbelkörper (2) verbindbar ist, ist vorgesehen, dass das Implantat (3) an seiner Oberseite bzw. seinem Oberteil (5) aus einem Material gebildet ist, welches sich von dem Material der Unterseite bzw. des Unterteils (4) des Implantats (3) unterscheidet und eine Gleitfläche der Oberseite (5) des Implantats (3) relativ zu dem daran anschließenden Wirbelkörper (2) ausbildet bzw. bereitstellt, wodurch sich eine physiologisch korrekte Beweglichkeit zwischen benachbarten Wirbelkörpern (1, 2) bereitstellen lässt.

Darüber hinaus wird ein Bausatz zur Herstellung eines Implantats zur Verfügung gestellt.

## Beschreibung

Die vorliegende Erfindung bezieht sich auf ein Implantat zum Einsetzen zwischen Wirbelkörper der Wirbelsäule, wobei das Implantat eine gewölbte Oberfläche an seiner Oberseite aufweist und in implantiertem Zustand an seiner Unterseite unbeweglich mit dem daran anschließenden Wirbelkörper verbindbar ist, sowie auf einen Bausatz zur Herstellung eines derartigen Implantats.

Eine Bandscheibe bzw. Zwischenwirbelscheibe zwischen Wirbelkörpern der Wirbelsäule soll üblicherweise eine Beweglichkeit benachbarter Wirbel relativ zueinander und eine Stoßdämpferwirkung zur Verfügung stellen. Aus verschiedenen medizinischen Gründen kann eine Entfernung einer derartigen Bandscheibe nötig sein, so dass nach Entfernung einer derartigen Bandscheibe ein Implantat zwischen Wirbelkörper der Wirbelsäule eingesetzt wird, wofür teilweise unterschiedliche Ausführungsformen bekannt sind.

Ein derartiges Implantat kann beispielsweise aus einem einzigen Stück bestehen, welches gegebenenfalls elastische Eigenschaften aufweist und mit den daran anschließenden Wirbelkörpern nicht verbunden ist. Ein derartiges Implantat gewährleistet eine reduzierte Beweglichkeit und gegebenenfalls eine gewisse Stoßdämpferwirkung, wobei derartige Implantate mit elastischen Eigenschaften beispielsweise aus der WO 94/23671, WO 93/16664, DE 37 41 493 A1, US-A 5 047 055, US-A 5 108 438, US-A 5 192 326, EP-A 0 304 305 oder der US-A 5 320 644 bekannt sind.

Gemäß einer abgewandelten Ausführungsform kann ein derartiges Implantat aus einem einzigen Stück bestehen, welches aus harten Deckplatten und einem elastischen Kern besteht, wobei die Deckplatten gegebenenfalls Verbindungs- oder Verankerungsmittel gegenüber den daran anschließenden Wirbelkörpern aufweisen. Derartige Implantate gewährleisten eine reduzierte Beweglichkeit und eine gewisse Stoßdämpferwirkung, wobei in diesem Zusammenhang beispielsweise auf die CH 671 691 A5, WO 90/11740, US-A 4 911 718, US-A 5 071 437, US-A 5 171 281 oder EP 0 346 269 A2 verwiesen wird.

Ein weiteres Implantat ist beispielsweise fix mit beiden Wirbelkörpern verbunden und besteht aus mindestens zwei Teilen, die beweglich miteinander verbunden sind, wie dies beispielsweise der FR-A 2 718 635 zu entnehmen ist. Jeder Teil ist mit einem Wirbelkörper fest verbunden. Zwischen diesen Teilen können elastische Elemente eingebaut sein. Diese zwei Teile gewährleisten eine reduzierte Beweglichkeit und gegebenenfalls eine gewisse Stoßdämpferwirkung.

Da derartige Implantate wenigstens teilweise elastische Eigenschaften aufweisen, wirken sie üblicherweise wie Stoßdämpfer bzw. flexible Abstandhalter. Insbesondere durch eine feste Verbindung mit den benachbarten bzw. daran anschließenden Wirbeln wird jedoch diesem Bereich der Wirbelsäule eine vom Design des Implantats vorbestimmte Beweglichkeit mit jeweils unphysiologischem Drehpunkt aufgezwungen.

Insbesondere im Bereich der Halswirbelsäule ist in einem derartigen Segment einer Bewegung zwischen benachbarten Wirbelkörpern ein Drehpunkt kein Punkt sondern eine Fläche, welche durch den Radius der Gelenksfortsätze und den Abstand bzw. die Höhe der Bandscheibe und die Wölbung im Zwischenwirbelraum vorgegeben wird, wobei sich der Drehpunkt innerhalb des Zwischenwirbelraums bewegt. Physiologisch korrekt wäre somit die Einräumung der Möglichkeit einer Bewegung benachbarter Wirbelkörper gegeneinander, wobei sich dies jedoch mit bekannten Implantaten nicht erzielen lässt.

Die vorliegende Erfindung zielt daher darauf ab, ein Implantat zum Einsetzen zwischen Wirbelkörper der Wirbelsäule zur Verfügung zu stellen, bei welchem die oben genannten Nachteile vermieden werden und insbesondere eine physiologisch korrekte Bewegung von Teilbereichen der Wirbelsäule, in welchen ein derartiges Implantat eingesetzt wird, zur Verfügung gestellt wird.

Zur Lösung dieser Aufgaben ist ein Implantat der eingangs genannten Art im Wesentlichen dadurch gekennzeichnet, dass das Implantat an seiner Oberseite bzw. seinem Oberteil aus einem Material gebildet ist, welches sich von dem Material der Unterseite bzw. des Unterteils des Implantats unterscheidet und eine Gleitfläche der Oberseite des Implantats relativ zu dem daran anschließenden Wirbelkörper ausbildet bzw. bereitstellt. Dadurch, dass erfindungsgemäß das Implantat aus Teilbereichen oder Teilen bzw. Elementen aus unterschiedlichen Materialien hergestellt ist, wird es möglich, das Implantat an seiner Unterseite bzw. seinem Unterteil für eine ordnungsgemäße Positionierung mit dem daran anschließenden Wirbelkörper zu verbinden, während die Oberseite bzw. das Oberteil des Implantats, welches aus einem von dem Material der Unterseite bzw. des Unterteils verschiedenen Material hergestellt ist, eine Beweglichkeit relativ zu dem daran anschließenden Wirbelkörper durch Bereitstellung einer Gleitfläche zur Verfügung stellt. Derart wird es möglich, eine physiologisch korrekte Beweglichkeit zwischen benachbarten Wirbelkörpern in dem Bereich der Wirbelsäule, in welchem das Implantat eingesetzt wird, zur Verfügung zu stellen.

Zur Bereitstellung der Gleitfläche der Oberseite bzw. des Oberteils zur Erzielung einer Beweglichkeit des an das eingesetzte Implantat an der Oberseite anschließenden Wirbelkörpers relativ zu dem Implantat sowie dem benachbarten bzw. an der Unterseite an das Implantat anschließenden Wirbelkörper wird gemäß einer bevorzugten Ausführungsform vorgeschlagen, dass die Oberseite bzw. das Oberteil des Implantats aus einem glatten abriebarmen Material gebildet ist.

Für eine ordnungsgemäße Verbindung bzw. Fixierung des Implantats an seiner Unterseite bzw. seinem Unterteil wird gemäß einer weiters bevorzugten Ausführungsform vorgeschlagen, dass die Unterseite bzw. das Unterteil des Implantats aus einem ein Einwachsen in den benachbarten Wirbelkörper unterstützenden Material gebildet ist.

Zur Bereitstellung eines einfach einbaubaren Implantats, welches erfindungsgemäß an seiner Oberseite bzw. seinem Oberteil und seiner Unterseite bzw. seinem Unterteil aus unterschiedlichen Materialien hergestellt ist, wird darüber hinaus vorgeschlagen, dass das Implantat im Wesentlichen entlang einer mittigen Trennfläche unterteilt ausgebildet ist, wobei das die Oberseite aufweisende Oberteil aus dem die Gleitfläche bildenden Material hergestellt ist und das die Unterseite aufweisende Unterteil mit dem daran anschließenden Wirbelkörper verbindbar ist, wie dies einer weiters bevorzugten Ausführungsform des erfindungsgemäßen Implantats entspricht. Derart lassen sich aus den unterschiedlichen Materialien die einzelnen Elemente, welche in weiterer Folge das Oberteil und das Unterteil des Implantats bilden, entsprechend zuverlässig und unter Erzielung bzw. Einhaltung der gewünschten Materialeigenschaften herstellen, so dass die angestrebte Beweglichkeit zwischen benachbarten Wirbelkörpern nach Einsetzen des Implantats zur Verfügung gestellt werden kann.

Zur Bereitstellung der Gleitfläche des Oberteils bzw. der Oberseite wird gemäß einer weiters bevorzugten Ausführungsform vorgeschlagen, dass die Oberseite bzw. das Oberteil aus einem Keramikmaterial hergestellt ist.

Zur Ermöglichung der zuverlässigen Verbindung der Unterseite bzw. des Unterteils des Implantats mit dem daran anschließenden Wirbelkörper wird gemäß einer weiters bevorzugten Ausführungsform vorgeschlagen, dass die Unterseite bzw. das Unterteil aus einem Metall, beispielsweise Titan, oder einem medizinischen Kunststoff, beispielsweise PEEK hergestellt ist.

Für eine zuverlässige Verbindung zwischen dem Oberteil und dem Unterteil des Implantats wird darüber hinaus vorgeschlagen, dass das Oberteil und das Unterteil über zueinander komplementäre Profilierungen, wie beispielsweise zueinander komplementäre Fortsätze bzw. Vorsprünge und Ausnehmungen bzw. Vertiefungen miteinander verbindbar sind, wie dies einer weiters bevorzugten Ausführungsform des erfindungsgemäßen Implantats entspricht.

Zur weiteren Unterstützung bzw. zur Bereitstellung einer sicheren Verbindung zwischen dem Oberteil und dem Unterteil des Implantats wird darüber hinaus bevorzugt vorgeschlagen, dass das Oberteil und das Unterteil durch eine Presspassung miteinander verbindbar sind.

Eine derartige Verbindung kann darüber hinaus dadurch unterstützt werden, dass das Oberteil und das Unterteil miteinander durch eine Verklebung verbindbar sind, wie dies einer weiters bevorzugten Ausführungsform des erfindungsgemäßen Implantats entspricht.

Zur Unterstützung eines Einwachsens in den benachbarten Wirbelkörper bzw. einer Verbindung des Implantats mit demselben wird darüber hinaus gemäß einer weiters bevorzugten Ausführungsform vorgeschlagen, dass die Unterseite bzw. das Unterteil in an sich bekannter Weise mit einer porösen Oberfläche und/oder mit einer Profilierung, insbesondere einer Mehrzahl von Erhebungen und Vertiefungen ausgebildet ist.

Eine derartige Verankerung bzw. Festlegung der Unterseite bzw. des Unterteils lässt sich gemäß einer weiters bevorzugten Ausführungsform dadurch unterstützen bzw. begünstigen, dass die Unterseite bzw. das Unterteil wenigstens einen Fortsatz bzw. Vorsprung zur Verankerung in dem dazu benachbarten Wirbelkörper aufweist.

Zur weiteren Unterstützung der Fixierung des Implantats an seiner Unterseite bzw. seinem Unterteil und/oder gegebenenfalls für eine provisorische Festlegung desselben wird darüber hinaus bevorzugt vorgeschlagen, dass das Unterteil über eine zusätzliche Fixierungs- bzw. Festlegungsvorrichtung, wie beispielsweise eine Schraube, einen Nagel, einen Dorn oder einen Anker, an dem dazu benachbarten Wirbelkörper festlegbar ist.

Unter Berücksichtigung der üblichen Ausbildung des Zwischenwirbelraums zwischen benachbarten Wirbelkörpern nach einer Entfernung der Bandscheibe und somit in Anpassung an übliche physiologische Gegebenheiten wird gemäß einer weiters bevorzugten Ausführungsform vorgeschlagen, dass das Implantat an seinen Enden eine gegenüber dem mittigen Bereich verringerte Höhe aufweist.

Die angestrebte Beweglichkeit zwischen benachbarten Wirbelkörpern unter Berücksichtigung physiologischer Gegebenheiten wird gemäß einer weiters bevorzugten Ausführungsform insbesondere dadurch unterstützt, dass die Höhe des Implantats an dem in eingebautem Zustand zum Dornfortsatz gewandten Ende geringer als die Höhe an dem zum Inneren der Wirbelkörper gewandten Ende ist.

Während somit das Implantat in einer Richtung von vorne nach hinten in eingebautem Zustand eine ungleichmäßige Krümmung und/oder Formgebung, beispielsweise durch Vorsehen unterschiedlicher Dicken bzw. Höhen aufweist, ist gemäß einer weiters bevorzugten Ausführungsform vorgesehen, dass das Implantat in eingebautem Zustand in lateraler Richtung eine symmetrische Krümmung aufweist.

Insbesondere zur Vermeidung einer Bereitstellung einer überaus großen Vielzahl von Kombinationen von Oberteilen und Unterteilen beispielsweise in Anpassung an eine Anordnung an unterschiedlichen Stellen der Wirbelsäule und/oder unter Berücksichtigung unterschiedlicher physiologischer Gegebenheiten wird darüber hinaus erfindungsgemäß ein Bausatz zur Herstellung eines Implantats der eingangs genannten Art oder einer bevorzugten Ausführungsform davon zur Verfügung gestellt, wobei der Bausatz insbesondere dadurch gekennzeichnet ist, dass eine Mehrzahl von Elementen unterschiedlicher Abmessungen und/oder unterschiedlicher Formgebung, insbesondere unterschiedlicher Höhe bzw. Dicke und/oder unterschiedlicher Krümmung bereitgestellt ist, wobei das Implantat aus jeweils einem Oberteil und einem Unterteil, welche aus der Mehrzahl von Elementen auswählbar sind, herstellbar bzw. zusammenbaubar ist. Durch Bereitstellung einer Mehrzahl von Elementen unterschiedlicher Abmessungen und/oder unterschiedlicher Formgebung wird es somit möglich, beispielsweise unmittelbar während der Operation und somit in Kenntnis der physiologischen Gegebenheiten nach einer Entfernung der Bandscheibe angepasst an die Raum- bzw. Platzverhältnisse sowie die Erfordernisse für die Bereitstellung der angestrebten Beweglichkeit Oberteile und Unterteile mit entsprechenden Abmessungen und/oder Formgebung, beispielsweise unterschiedlicher Höhe bzw. Dicke und/oder unterschiedlicher Krümmung zu einem aus einem Oberteil oder einem Unterteil bestehenden Implantat zusammenzusetzen und gegebenenfalls miteinander zu verbinden, wie dies oben bereits ausgeführt wurde. Sollte eine derartige Zusammenstellung bzw. ein derartiger Zusammenbau von einzelnen Elementen eines Implantats während eines Operationsvorgangs nicht für sinnvoll erachtet werden, so ermöglicht die Bereitstellung des erfindungsgemäßen Bausatzes zur Herstellung eines Implantats unter Einsatz von Elementen unterschiedlicher Abmessungen und/oder Formgebung eine entsprechend vereinfachte Herstellung der einzelnen Elemente, welche nachfolgend auch vorbereitend für die Durchführung einer Operation zu unterschiedlichen Implantaten zusammengesetzt bzw. zusammengebaut werden können, welche eine Anpassung an unterschiedliche physiologische Gegebenheiten ermöglichen.

Die Erfindung wird nachfolgend anhand von der in der beiliegenden Zeichnung schematisch dargestellten Ausführungsbeispielen näher erläutert. In dieser zeigen:
Fig. 1 eine schematische Seitenansicht von zwei benachbarten Wirbelkörpern, zwischen welche nach Entfernung einer Bandscheibe ein erfindungsgemäßes Implantat eingesetzt ist;
Fig. 2 eine Rückansicht auf die in Fig. 1 dargestellten Wirbelkörper mit einem eingesetzten erfindungsgemäßen Implantat gemäß dem Pfeil II von Fig. 1, wobei Fig. 1 eine Ansicht in Richtung des Pfeils I von Fig. 2 darstellt;
Fig. 3 eine Seitenansicht auf eine abgewandelte Ausführungsform eines Implantats ähnlich der Darstellung von Fig. 1, wobei die Wirbelkörper der Einfachheit halber nicht dargestellt sind;
Fig. 4 eine Ansicht ähnlich der Position von Fig. 2 des Implantats von Fig. 3; und
Fig. 5 in einer zu Fig. 3 ähnlichen Darstellung eine Seitenansicht einer weiteren abgewandelten Ausführungsform eines erfindungsgemäßen Implantats.

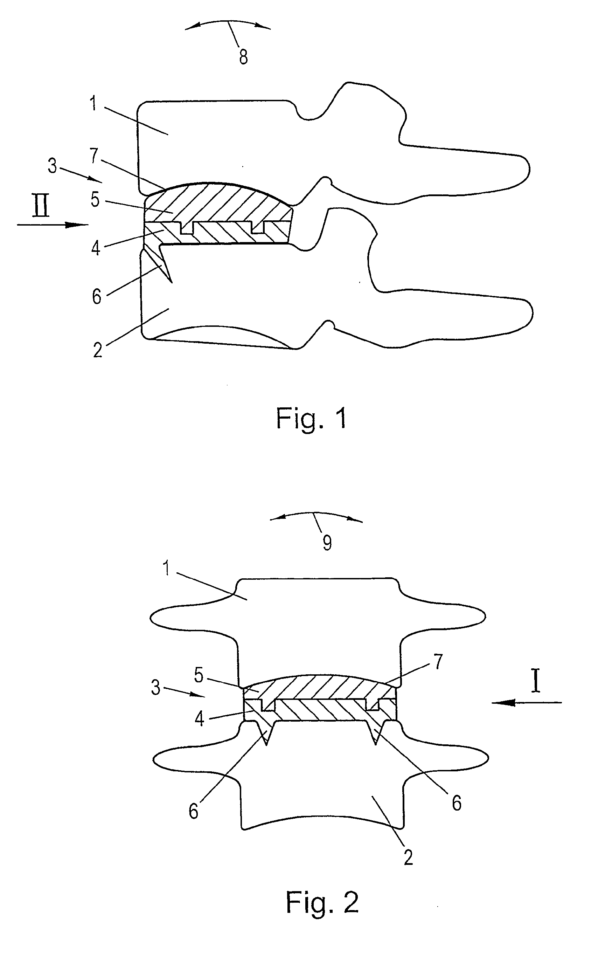

In Fig. 1 und 2 sind schematisch mit 1 und 2 zwei benachbarte Wirbelkörper bezeichnet, zwischen welche nach Entfernung einer Bandscheibe ein allgemein mit 3 bezeichnetes Implantat eingesetzt ist. Wie dies nachfolgend noch im Detail erötertert werden wird, besteht das Implantat 3 aus einem Unterteil 4 und einem Oberteil 5, wobei das Unterteil 4 mit dem darunterliegenden bzw. daran anschließenden Wirbelkörper 2 verbindbar ist. In der in Fig. 1 und 2 dargestellten Ausführungsform sind für ein Festlegen des Unterteils 4 an dem daran anschließenden Wirbelkörper 2 dornförmige Fortsätze 6 angedeutet. Diese Fortsätze bzw. Vorsprünge 6 werden im Knochenmaterial des daran anschließenden Wirbelkörpers 2 verankert.

Neben dem Unterteil 4 weist das Implantat ein damit verbundenes bzw. verbindbares Oberteil 5 auf, welches, wie dies ebenfalls nachfolgend erörtert werden wird, eine glatte Oberfläche zur Bereitstellung einer Gleitfläche 7 relativ zu dem daran anschließenden Wirbelkörper 1 zur Verfügung stellt. Durch Bereitstellung der Gleitfläche 7 wird es möglich, entsprechend den physiologischen Gegebenheiten eine Bewegbarkeit zwischen den benachbarten Wirbelkörpern 1 und 2 entsprechend den Doppelpfeilen 8 und 9 in im Wesentlichen normal aufeinander stehenden Richtungen sowie eine Rotationsbewegung zwischen den benachbarten Wirbelkörpern zur Verfügung zu stellen.

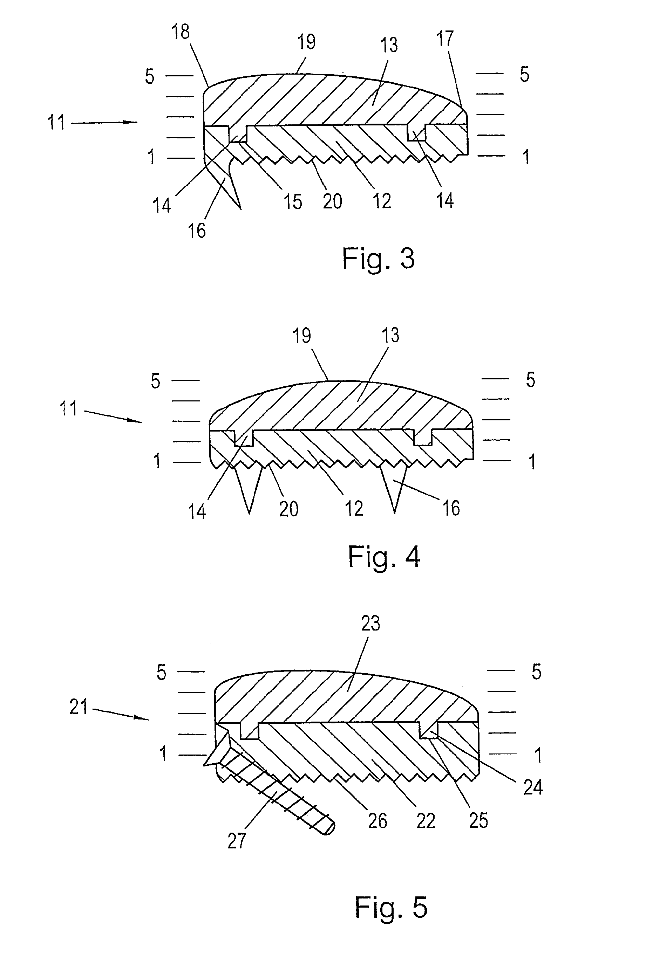

In Fig. 3 und 4 ist ein mit dem Implantat 3 gemäß Fig. 1 und 2 vergleichbares Implantat 11 dargestellt, welches jeweils wiederum aus einem Unterteil 12 und einem Oberteil 13 zusammengesetzt ist.

Für eine Verbindung zwischen dem Oberteil 13 und dem Unterteil 12 weist in der dargestellten Ausführungsform das Oberteil 13 eine Mehrzahl von Fortsätzen bzw. Vorsprüngen 14 auf, welche in entsprechende komplementäre Ausnehmungen 15 des Unterteils 12 einsetzbar sind.

Bei Vorsehen einer Presspassung zwischen dem Oberteil 13 und dem Unterteil 12 kann unmittelbar eine sichere und zuverlässige Verbindung zwischen diesen beiden Teilen bzw. Elementen 12 und 13 zur Verfügung gestellt werden.

Für eine Verankerung des Unterteils 12 in einem daran anschließenden, in Fig. 3 und 4 nicht näher dargestellten Wirbelkörper sind ähnlich wie bei der Ausführungsform gemäß Fig. 1 und 2 keilartige Fortsätze 16 vorgesehen.

Weiters ist aus der Darstellung gemäß Fig. 3 ersichtlich, dass das Implantat 11 und insbesondere das Oberteil 13 in der Seitenansicht an seinem in eingebautem Zustand zum Dornfortsatz gewandten Ende 17 mit einer geringeren Höhe als an dem zum Inneren der Wirbelkörper gewandten Ende 18 ausgebildet ist.

In Fig. 3 ist zusätzlich schematisch eine Skala mit willkürlichen Einheiten von 1 bis 5 angedeutet, woraus ersichtlich ist, dass das Implantat 11 an seinen Endbereichen 17 und 18 eine unterschiedliche Höhe und somit insgesamt an seiner Oberfläche 19 eine asymmetrische Krümmung bzw. Formgebung aufweist.

Aus der Darstellung gemäß Fig. 4 ist ersichtlich, dass in einer lateralen Richtung das Implantat 11 und insbesondere das Oberteil 13 an der Oberfläche 19 eine symmetrische Krümmung bzw. Formgebung aufweist.

Unter Berücksichtigung der angegebenen Skalierung ist darüber hinaus ersichtlich, dass unter Verwendung von Teilen bzw. Elementen 12 und 13 unterschiedlicher Dicke bzw. Höhe derart auch ein Implantat 11 mit unterschiedlicher Gesamthöhe bzw. -dicke bereitgestellt werden kann. In ähnlicher Weise kann durch entsprechende Wahl unterschiedlicher Formgebungen bzw. Krümmungen eine Anpassung an unterschiedliche physiologische Gegebenheiten erzielt werden.

Zur Unterstützung der Verankerung in dem daran anschließenden Wirbelkörper weist darüber hinaus die Ausbildung des Implantats 11 gemäß Fig. 3 und 4 eine Profilierung 20 des Unterteils 12, insbesondere eine Mehrzahl von Erhebungen und Vertiefungen auf, welche ein Einwachsen in das Material des daran anschließenden Wirbelkörpers unterstützen.

Anstelle oder zusätzlich zu einer derartigen Profilierung 20 kann die zum Wirbelkörper gewandte Oberfläche des Unterteils 12 porös ausgebildet sein, um derart ebenso ein Einwachsen bzw. Anwachsen des Unterteils 12 in bzw. an den daran anschließenden Wirbelkörper nach einem Einsetzen des Implantats 11 zu begünstigen.

Demgegenüber ist die Oberfläche 19 des Oberteils 13 als Gleitfläche aus einem glatten abriebarmen Material zur Aufrechterhaltung der Bewegbarkeit ausgebildet, wie dies in Fig. 1 und 2 insbesondere durch die Doppelpfeile 8 und 9 angedeutet ist.

In Fig. 5 ist eine Seitenansicht ähnlich zu der Darstellung gemäß Fig. 3 einer weiteren abgewandelten Ausführungsform eines Implantats 21 dargestellt. Das Implantat 21 besteht wiederum aus einem Unterteil 22 und einem Oberteil 23. Ähnlich wie bei der Ausführungsform gemäß Fig. 3 und 4 erfolgt eine Verbindung zwischen dem Oberteil 23 und dem Unterteil 22 über komplementäre Vorsprünge bzw. Fortsätze 24 und entsprechende Vertiefungen 25.

Zur Unterstützung der Fixierung des Unterteils 22 in dem wiederum nicht dargestellten daran anschließenden Wirbelkörper in eingebautem Zustand weist das Unterteil 22 neben einer mit einer Profilierung versehenen Oberfläche 26 die Möglichkeit der Anordnung einer Fixierungs- bzw. Festlegungsvorrichtung, wie beispielsweise einer Schraube 27 auf, wobei über diese wenigstens eine Schraube 27 eine unmittelbare Verankerung in einem daran anschließenden Wirbelkörper vorgenommen wird. Anstelle einer derartigen Schraube kann auch ein Nagel, ein Dorn oder ein Anker zum Einsatz gelangen.

Anstelle oder zusätzlich zu den dargestellten komplementären Fortsätzen und Ausnehmungen 14 und 15 bzw. 24 und 25 kann zur Kopplung der Teile des Implantats 11 bzw. 21 eine Verklebung zwischen dem jeweiligen Oberteil und Unterteil 13 und 12 bzw. 23 und 22 vorgesehen sein.

Zur Bereitstellung eines eine Gleitfläche ausbildenden bzw. darstellenden Oberteils 5, 13 und 23 ist dieses beispielsweise aus einem Keramikmaterial hergestellt, um derart eine entsprechend glatte und abriebarme Oberfläche zur Verfügung zu stellen.

Zur Unterstützung der Verbindung des Unterteils 4, 12 und 22 ist dieses beispielsweise aus einem Metall, beispielsweise Titan oder einem medizinischen Kunststoff, beispielsweise PEEK hergestellt.

Durch Verwendung dieser Materialien lassen sich in einfacher und zuverlässiger Weise Implantate 3, 11 bzw. 21 herstellen, wobei neben einer sicheren Verankerung des Unterteils 4, 12 oder 22 an einem daran anschließenden Wirbelkörper auch eine entsprechend abriebarme Gleitfläche des Oberteils 5, 13 oder 23 zur Erhaltung der Beweglichkeit zwischen den Wirbelkörpern 1 und 2 zur Verfügung gestellt wird.

Bei Bereitstellung eines Bausatzes zur Zusammenstellung unterschiedlicher Oberteile 5, 13 oder 23 und Unterteile 4, 12 oder 22 mit unterschiedlichen Abmessungen, insbesondere unterschiedlicher Dicke bzw. Höhe, und/oder unterschiedlicher Formgebung, insbesondere unterschiedlicher Krümmung, wie dies beispielsweise aus Fig. 3 bis 5 deutlich ersichtlich ist, lassen sich Implantate 3, 11 und 21 zur Verfügung stellen, welche in einfacher und zuverlässiger Weise an die physiologischen Gegebenheiten anpassbar sind.

Während bei den dargestellten Ausführungsformen jeweils das Oberteil 13 bzw. 23 mit entsprechenden Fortsätzen bzw. Erhebungen 14 bzw. 24 ausgebildet ist, welche in komplementäre Vertiefungen 15 bzw. 25 eintreten, kann eine zuverlässige Positionierung bzw. Verbindung zwischen den einzelnen Elementen 12 und 13 bzw. 22 und 23 durch Vorsehen entsprechender Erhebungen bzw. Fortsätze an den Unterteilen 12 bzw. 22 und entsprechenden komplementären Vertiefungen an den Oberteilen 13 bzw. 23 zur Verfügung gestellt werden.

Anstelle einer getrennten Ausbildung eines Oberteils 5, 13, 23 und eines Unterteils 4, 12, 22 eines Implantats 3, 11, 21 mit voneinander verschiedenen Materialeigenschaften kann ein derartiges Implantat auch aus einem Stück bzw. Element ausgebildet sein, welches an seiner Oberseite bzw. einem in ein eingebauter Lage oberen Teilbereich von seiner Unterseite bzw. unterem Teilbereich verschiedene Materialeigenschaften ähnlich den oben erwähnten Oberteilen und Unterteilen aufweist.

## Patentansprüche

1. Implantat zum Einsetzen zwischen Wirbelkörper (1, 2) der Wirbelsäule, wobei das Implantat (3, 11, 21) eine gewölbte Oberfläche (7, 19) an seiner Oberseite aufweist und in implantiertem Zustand an seiner Unterseite unbeweglich mit dem daran anschließenden Wirbelkörper (2) verbindbar ist, **dadurch gekennzeichnet, dass** das Implantat (3, 11, 21) an seiner Oberseite bzw. seinem Oberteil (5, 13, 23) aus einem Material gebildet ist, welches sich von dem Material der Unterseite bzw. des Unterteils (4, 12, 22) des Implantats (3, 11, 21) unterscheidet und eine Gleitfläche der Oberseite (5, 13, 23) des Implantats (3, 11, 21) relativ zu dem daran anschließenden Wirbelkörper (1) ausbildet bzw. bereitstellt.

2. Implantat nach Anspruch 1, **dadurch gekennzeichnet, dass** die Oberseite bzw. das Oberteil (5, 13, 23) des Implantats (3, 11, 21) aus einem glatten abriebarmen Material gebildet ist.

3. Implantat nach Anspruch 1 oder 2, **dadurch gekennzeichnet, dass** die Unterseite bzw. das Unterteil (4, 12, 22) des Implantats (3, 11, 21) aus einem ein Einwachsen in den benachbarten Wirbelkörper (2) unterstützenden Material gebildet ist.

4. Implantat nach Anspruch 1, 2 oder 3, **dadurch gekennzeichnet, dass** das Implantat (3, 11, 21) im Wesentlichen entlang einer mittigen Trennfläche unterteilt ausgebildet ist, wobei das die Oberseite aufweisende Oberteil (5, 13, 23) aus dem die Gleitfläche bildenden Material hergestellt ist und das die Unterseite aufweisende Unterteil (4, 12, 22) mit dem daran anschließenden Wirbelkörper (2) verbindbar ist.

5. Implantat nach einem der Ansprüche 1 bis 4, **dadurch gekennzeichnet, dass** die Oberseite bzw. das Oberteil (5, 13, 23) aus einem Keramikmaterial hergestellt ist.

6. Implantat nach einem der Ansprüche 1 bis 5, **dadurch gekennzeichnet, dass** die Unterseite bzw. das Unterteil (4, 12, 22) aus einem Metall, beispielsweise Titan, oder einem medizinischen Kunststoff, beispielsweise PEEK hergestellt ist.

7. Implantat nach einem der Ansprüche 1 bis 6, **dadurch gekennzeichnet, dass** das Oberteil (5, 13, 23) und das Unterteil (4, 12, 22) über zueinander komplementäre Profilierungen, wie beispielsweise zueinander komplementäre Fortsätze bzw. Vorsprünge (14, 24) und Ausnehmungen bzw. Vertiefungen (15, 25) miteinander verbindbar sind.

8. Implantat nach einem der Ansprüche 1 bis 7, **dadurch gekennzeichnet, dass** das Oberteil (5, 13, 23) und das Unterteil (4, 12, 22) durch eine Presspassung miteinander verbindbar sind.

9. Implantat nach einem der Ansprüche 1 bis 8, **dadurch gekennzeichnet, dass** das Oberteil (5, 13, 23) und das Unterteil (4, 12, 22) miteinander durch eine Verklebung verbindbar sind.

10. Implantat nach einem der Ansprüche 1 bis 9, **dadurch gekennzeichnet, dass** die Unterseite bzw. das Unterteil (12, 22) in an sich bekannter Weise mit einer porösen Oberfläche und/oder mit einer Profilierung (20, 26), insbesondere einer Mehrzahl von Erhebungen und Vertiefungen ausgebildet ist.

11. Implantat nach einem der Ansprüche 1 bis 10, **dadurch gekennzeichnet, dass** die Unterseite bzw. das Unterteil (4, 12) wenigstens einen Fortsatz bzw. Vorsprung (6, 16) zur Verankerung in dem dazu benachbarten Wirbelkörper (2) aufweist.

12. Implantat nach einem der Ansprüche 1 bis 11, **dadurch gekennzeichnet, dass** das Unterteil (22) über eine zusätzliche Fixierungs- bzw. Festlegungsvorrichtung (27), wie beispielsweise eine Schraube, einen Nagel, einen Dorn oder einen Anker, an dem dazu benachbarten Wirbelkörper (2) festlegbar ist.

13. Implantat nach einem der Ansprüche 1 bis 12, **dadurch gekennzeichnet, dass** das Implantat (3, 11, 21) an seinen Enden eine gegenüber dem mittigen Bereich verringerte Höhe aufweist.

14. Implantat nach einem der Ansprüche 1 bis 13, **dadurch gekennzeichnet, dass** die Höhe des Implantats (11) an dem in eingebautem Zustand zum Dornfortsatz gewandten Ende (17) geringer als die Höhe (18) an dem zum Inneren der Wirbelkörper (1, 2) gewandten Ende ist.

15. Implantat nach einem der Ansprüche 1 bis 14, **dadurch gekennzeichnet, dass** das Implantat (3, 11, 21) in eingebautem Zustand in lateraler Richtung eine symmetrische Krümmung aufweist.

16. Bausatz zur Herstellung eines Implantats nach einem der Ansprüche 1 bis 15, **dadurch gekennzeichnet, dass** eine Mehrzahl von Elementen unterschiedlicher Abmessungen und/oder unterschiedlicher Formgebung, insbesondere unterschiedlicher Höhe bzw. Dicke und/oder unterschiedlicher Krümmung bereitgestellt ist, wobei das Implantat (3, 11, 21) aus jeweils einem Oberteil (5, 13, 23) und einem Unterteil (4, 12, 22), welche aus der Mehrzahl von Elementen auswählbar sind, herstellbar bzw. zusammenbaubar ist.
